Europäisches Patentamt

European Patent Office

Office européen des brevets

(11) Publication number: **0 287 652 B1**

# EUROPEAN PATENT SPECIFICATION

(45) Date of publication of patent specification: **12.01.94**  (51) Int. Cl.⁵: **C12N 5/00**, C12N 5/02

(21) Application number: **87907471.4**

(22) Date of filing: **20.10.87**

(86) International application number:
**PCT/US87/02721**

(87) International publication number:
**WO 88/02774 (21.04.88 88/09)**

(54) **SERUM-FREE MEDIUM FOR THE PROLIFERATION OF LYMPHOKINE-ACTIVATED KILLER CELLS.**

(30) Priority: **20.10.86 US 920387**

(43) Date of publication of application:
**26.10.88 Bulletin 88/43**

(45) Publication of the grant of the patent:
**12.01.94 Bulletin 94/02**

(84) Designated Contracting States:
**AT BE CH DE FR GB IT LI LU NL SE**

(56) References cited:

**JOURNAL OF IMMUNOLOGICAL METHODS, vol. 86, 1986, North-Holland, Amsterdam (NL); C.J. FROELICH et al, pp. 205-211&NUM;**

**JOURNAL OF BIOLOGICAL CHEMISTRY, vol. 260, no. 3, 10 February 1985, American Society for Biochemistry and Molecular Biology, Baltimore, MD (US); K. KAIBUCHI et al., pp. 1366-1369&NUM;**

(73) Proprietor: **LIFE TECHNOLOGIES INC.**
**8717 Grovemont Circle,**
**P.O. Box 6009**
**Gaithersburg, MD 20877(US)**

(72) Inventor: **DARFLER, Frederick, J.**
**7000 Needwood Road**
**Derwood, MD 20855(US)**

(74) Representative: **Sheard, Andrew Gregory et al**
**Kilburn & Strode**
**30, John Street**
**London WC1N 2DD (GB)**

EP 0 287 652 B1

ANIMAL CELL BIOTECHNOLOGY, vol. 1, 1985, Academic Press, New York,NY (US); LAMBERT et al., pp. 85-122&NUM;

CANCER IMMUNOLOGY IMMUNOTHERAPY, vol. 21, 1986, Springer, New York, NY (US); SHIIBA et al., pp. 119-128&NUM;

JOURNAL OF BIOLOGICAL CHEMISTRY, vol. 261, no. 15, 25 May 1986, Baltimore, MD (US); BISHOP et al., pp. 6993-6999&NUM;

JOURNAL OF BIOLOGICAL CHEMISTRY, vol. 260, no. 3, 10 February 1985, Baltimore, MD (US); LAPETINA et al., pp. 1358-1361&NUM;

BIOCHEMICAL AND BIOPHYSICAL RESEARCH COMMUNICATIONS, 30 August 1985, Academic Press, New York, NY (US); GUY et al., pp. 484-491&NUM;

NATURE, vol. 315, 16 May 1985, MacMillan, London (GB); FARRAR et al., pp. 233-235&NUM;

JOURNAL OF BIOLOGICAL CHEMISTRY, vol. 262, 05 April 1987, Baltimore, MD (US); KOYASU et al., pp. 4689-4695&NUM;

PROCEEDINGS OF THE NATL. ACADEMY OF SCIENCES USA, vol. 82, February 1985, Washington, DC (US); EBELING et al., pp. 815-819&NUM;

JOURNAL OF EXPERIMENTAL MEDICINE, vol. 155, June 1982, New York, NY (US); GRIMM et al., pp. 1823-1841&NUM;

CANCER RESEARCH, vol. 44, May 1984, Waverly Press Inc., Baltimore, MD (US); ROSENSTEIN et al., pp. 1946-1953&NUM;

CANCER TREATMENT REPORTS, vol. 68, no. 1, January 1984, Government Printing Office, Bethesda, MD (US); ROSENBERG et al., pp. 233-255&NUM;

JOURNAL OF EXPERIMENTAL MEDICINE, vol. 159, February 1984, Rockefeller Union Press, New York, NY (US); MAZUMDER et al., pp. 495-507&NUM;

ANALYTICAL BIOCHEMISTRY, vol. 102, 1980, Academic Press, New York,NY (US); BARNESS et al., pp. 255-270&NUM;

NATURE, vol. 313, 24 January 1985, MacMillan Magazines, London (GB); TRUNEH et al., pp. 318-320&NUM;

JOURNAL OF EXPERIMENTAL MEDICINE, Rockefeller Union Press, New York, NY (US), vol. 158, October 1983; GRIMM et al., pp. 1356-1361&NUM;

## Description

FIELD OF THE INVENTION

The invention pertains to compositions suitable for the in vitro culturing of lymphokine-activated killer cells. Specifically, the invention discloses a serum-free medium capable of permitting the proliferation of such cells.

BACKGROUND ART

One of the most promising approaches in the treatment of cancer involves the concept of passive immunotherapy. This approach involves the transfer to a cancer patient of previously sensitized immunologic reagents, such as cells or antibodies, that have the ability to provoke an anti-tumor response (Rosenberg, S.A., Canc. Treat. Rep., 68:233-255 (1984)). Among the many possible immunologic reagents which may be administered, are lymphokines, such as interleukin-2 (IL-2) or interferon. Unfortunately, cancer patients are typically immunosuppressed, and consequently, the success of immunotherapy has been more limited than had been hoped (Rosenberg (1984)). Recently, Grimm and co-workers identified a novel approach to immunotherapy which has significant potential for overcoming this drawback (Grimm, E.A., et al., J. Ever. Med., 155:1823-1841 (1982); Grimm, E.A., et al., J. Exper. Med., 157:884-897 (1983); Grimm, E.A., et al., J. Exper. Med., 158:1356-1361 (1983)). These researchers discovered that if peripheral blood lymphocyte (PBL) cells were removed from a patients and incubated in vitro in the presence of interleukin-2, the cells would acquire the ability to preferentially recognize and destroy tumor cells. This discovery is especially significant in view of the immunosuppressed state of typical cancer patients, in that it enables the physician to withdraw a small number of lymphocytes and to culture these cells in vitro to produce a large number of tumor-specific immunocompetent cells. Since these cells were activated by the presence of a lymphokine to kill tumor cells, they have been referred to as lymphokine activated killer cells (LAK) cells.

Although initial experiments were conducted using natural lymphokines, it has subsequently been discovered that lymphokines produced through recombinant DNA technology are also capable of mediating this activation (Rayner, A.A., et al., Cancer, 55:1327-1333 (1985), Itoh, K., et al., J. Immunol., 134:3124-3129 (1985), Mule, J.J., et al., J. Immunol., 135:646-652 (1985)).

Since PBL cells are naturally present in human blood and lymphatic tissue, it had initially been believed that the administration of a lymphokine to a cancer patient would result in the patient's generation of LAK cells. The generally immune suppressed nature of cancer patients, and the presence of specific factors which suppress LAK cell induction have, thus far, blocked this potential therapeutic regimen. Hence, in order to produce LAK cells, it is necessary to cultivate PBL cells in vitro, in the presence of a lymphokine, and then to administer the in vitro derived LAK cells to a cancer patient.

The in vitro generation of LAK cells was originally attempted in culture medium which contained human serum (Grimm, E.A., et al., J. Exper. Med., 157:884-887 (1983)). The extreme cost of such sera, its limited availability and the possibility that it might be contaminated by microorganisms, especially viruses prompted investigations aimed at identifying alternative culture media which would be capable of proliferating the PBL cells and the generation of LAK cells.

Mazumder, A., et al., (J. Exper. Med., 159:495-507 (1984)) and Rosentein, M. et al., (J. Natl. Canc. Inst., 72:1161-1165 (1984)) demonstrated that culture media which contained fetal calf sera was capable of stimulating the production of LAK cells. Fetal calf sera was, however, found to be significantly less effective than human sera in inducing LAK cell formation (Imir, P. et al., Clin. Immunol. Immunopath., 36:289-296 (1985)). These workers found that the number of LAK cells which could be generated in human sera was more than eight times higher than that which an equivalent PBL cell innoculum could produce when cultured in medium containing bovine sera. These results led Imir, et al. to conclude that fetal calf sera appeared to inhibit the generation of LAK cells, and thus that human sera was a preferred culture supplement. Moreover, the use of fetal calf sera appeared to have a direct toxic effect on either the LAK cell precursors or the LAK cells themselves (Imir, et al., (1985)).

In response to the above-described limitations, Froelich, C.J., et al. (J. Immunol. Meth., 86:205-211 (1986)) identified a serum-free medium capable of both supporting the proliferation of PBL and the generation of LAK cells. The medium identified by these researchers was Dulbecco's Modified Eagle's Medium which was supplemented with bovine serum albumin and the three fatty acids: linoleic acid, oleic acid and palmitic acid. The medium also contained insulin, transferrin, and ethanolamine. This medium was capable of permitting the proliferation of LAK cells, albeit at an efficiency significantly lower than that of human sera-containing media. (Froelich, C.J., et al., (1986)) found that the efficiency of LAK cell generation by this serum-free medium could be enhanced through the addition of sodium pyruvate and indomethacin, an inhibitor of prostaglandin biosynthesis. Even with these supplements, however, the serum-free medium described by Froelich, et al. was found to be significantly less effective than media containing human sera in permitting the proliferation of LAK cells (Froelich, et al. (1986)). In addition, the inclusion of bovine serum albumin to the serum-free medium raised questions as to whether an immune response would be mounted in vitro against the foreign bovine albumin and as a result, indirectly activate LAK cell expansion and activation. Moreover, the possibility that a patient might mount an immune response, in vivo, due to the presence of the foreign bovine albumin raised questions as to the suitability of using bovine albumin to culture PBL cells which would subsequently be returned to a patient.

The present invention is based upon the discovery that the activation of kinase C, a natural and endogenous enzyme present in PBL cells, permits PBL cells to develop into proliferating LAK cells.

Kinase C is an enzyme which has been found to be the physiologic receptor for phorbol esters, a unique class of tumor promoters (Farrar, W.L., et al., Lymphokine Res., 4:87-93 (1985)). Interleukin-2 has been found to be capable of activating the kinase C enzyme. Activated kinase C, has been found to be capable of phosphorylating the interleukin-2 receptor molecule of intact lymphocytes, suggesting that it may have a role in an animal's immune response (Farrar, et al. (1985) Taguchi, M., et al., Biochem. Biophys. Res. Commun., 135:239-247 (1986)).

Once activated by the lymphokine, LAK cells are capable of proliferating and producing gamma interferon. It is believed that the gamma interferon so produced, interacts in concert with the exogenously added interleukin-2 to further stimulate the production of gamma interferon. The presence of gamma interferon is further believed to be involved in the expression of cellular receptor sites for the interleukin-2 molecule (Shiiba, K., Cancer, Immunol. Immunother., 21:119-128 (1986)). The gamma interferon produced by LAK cells is believed to be responsible for the cytotoxic and therapeutic properties of the cells (Rosenstein, M., et al., Cancer Res. 44: 1946-1953 (1984); Rayner, A.A., et al., J. Natl. Canc. Inst. 75:67-74 (1985)).

Thus, in summary, the ability to treat cancer patients with the LAK cell therapy discovered by Grimm, et al., (1983) requires the use of a cell culture medium which is both economical and effective in permitting the generation of such cells. The use of human sera, though effective in generating LAK cells, is prohibitively expensive, a potential carrier of viruses and of limited supply. Efforts to employ alternative non-human sera or serum-free media have not proven to be wholly satisfactory. Thus, a continued need exists for an alternative, economical and effective culture for generating LAK cells.

## SUMMARY OF THE INVENTION

The present invention relates to methods and compositions of matter which permit the generation of LAK cells in a defined, serum-free culture medium. The cell culture medium permits the proliferation of LAK cells at an efficiency which is substantially equivalent to that obtainable through the use of a culture medium which contain human serum. The cell culture medium of the present invention is, however, superior to such serum-containing culture media in that the use of a defined, serum-free medium can facilitate the elucidation of the mechanism which regulates the activation of PBL cells into LAK cells.

In a first aspect the invention relates to a method of producing a lymphokine activated killer cell, the method comprising:

(a) contacting a peripheral blood lymphocyte (PBL) cell with an amount of a culture medium sufficient to permit the in vitro cultivation of the peripheral blood lymphocyte cell, the medium being substantially free of serum and containing:

(i) a lymphokine; and

(ii) a non-proteinaceous compound capable of activating the enzyme kinase C which is a diacylglycerol, a functional analogue of a diacylglycerol, or a precursor of a diacylglycerol, an acetylacylglycerol, a functional analogue of an acetylacylglycerol, or a precursor of an acetylacylglycerol; and

(b) maintaining the PBL cell in the culture medium under conditions sufficient to permit the production of the lymphokine activated killer cell.

A second aspect relates to lymphokine activated killer (LAK) cells prepared by the process of the first aspect.

A third aspect relates to the use of activated LAK cells prepared by the first aspect in the preparation of an agent for the suppression of growth or development of a cancer cell.

A fourth aspect of the invention is directed toward a composition of matter which comprises: (a) an in vitro cultivated peripheral blood lymphocyte cell or lymphokine activated killer cell, and (b) an exogenously added nonproteinaceous compound capable of activating the enzyme kinase C which is a diacylglycerol, a functional analogue of a diacylglycerol, or a precursor of a diacylglycerol, an acetylacylglycerol, a functional analogue of an acetylacylglycerol, or a precursor of an acetylacylglycerol; wherein the composition of matter is substantially free of serum.

Additionally, a fifth aspect of the invention pertains to a culture medium which contains a lymphokine and an exogenously added non-proteinaceous compound capable of activating the enzyme kinase C which is a diacylglycerol, a functional analogue of a diacylglycerol, or a precursor of a diacylglycerol, an acetylacylglycerol, a functional analogue of an acetylacylglycerol, or a precursor of an acetylacylglycerol; wherein the culture medium is substantially free of serum. The culture medium may additionally contain an agent capable of inhibiting breakdown of the non-proteinaceous compound, e.g. 1-monooleoyl-rac-glycerol, dioctanoylethyleneglycol, dioctanoyl glyceramide or dioctanoyl butanetriol. Such a non-proteinaceous compound might be acetylacylglycerol, a functional analogue of an acetylacylglycerol or a precursor of an acetylacylglycerol. Preferably the agent is an inhibitor of the enzyme diacylglycerol kinase or diacylglycerol lipase.

Preferred features, method and embodiments of one aspect of the invention are, where appropriate, also preferred for the other four aspects of the present invention.

## DESCRIPTION OF THE PREFERRED EMBODIMENTS

I. The In Vitro Cultivation of PBL Cells

A cell is said to be "maintained" in a tissue culture medium when it is cultivated under conditions sufficient to either maintain cellular viability or promote proliferative growth. As used herein, the term "cellular viability" refers to the ability of a cell to metabolize nutrients, and direct biosynthetic processes. The term "proliferative growth" refers to the ability of a cell to produce progeny cells. The maintenance of cellular viability does not require the proliferative growth of the cell.

In general, the in vitro cultivation of the cells of the invention requires a consideration of four basic elements: (1) the nature of the substrate or phase on or in which the cells are to be cultivated; (2) the composition and concentration of the gaseous atmosphere in which the cells will be incubated; (3) the temperature of incubation; and (4) the physiochemical and physiological constitution of the culture medium (Freshney, R.I., In: Culture of Animal Cells, a Manual of Basic Technique, Alan R. Liss, Inc. N.Y. pp55-78 (1983); Lambert, K.J., et al., In: Animal Cell BioTechnology, Vol. 1, Spier, R.E., et al., Eds., Acad. Press N.Y. pp86-122 (1985) which references are hereby incorporated by reference). Thus, important considerations include whether it is desirable to cultivate the cells in a suspension of culture medium or to permit them to grow in contact with a solid surface. Suitable solid surfaces include glass, polystyrene culture dishes, or the surfaces of suspendible particulates. Alternatively, the cells may be grown in permeable membranous spheres or in hollow fibers. Although any cultivation technique capable of maintaining the cellular viability or proliferative growth capacity of PBL cells may be employed, it is preferable to cultivate such cells in a suspension in T-flasks or roller bottles (Mazumder, A., et al., Cancer 53:896-905 (1984)), although other methods, such as growth in air-lift or deep tank fermenters or hollow fiber, ultra-filtrative devices, may also be suitable. The volume of culture medium may be as little as 50 $\mu$l or as much as 100 liters.

Although the cells may be incubated at any temperature in which cellular viability may be sustained, it is preferable to maintain cells at temperatures between 30 and 45°C and it is most preferable to cultivate the cells at an incubation temperature of 37°C. Cultured cells will, however, tolerate considerable drops in temperatures and can survive several days at 4°C and can be frozen and cooled to -196°C in the pressence of suitable cryopreservatives. In regulating the temperature of cultured cells, consistency of temperature maintenance is important; the temperature should be maintained within ± 2°C of the desired temperature.

The gaseous atmosphere in which the cells are incubated must contain oxygen, and carbon dioxide. Although numerous gaseous compositions and concentrations are possible, it is preferable to employ a gaseous atmosphere containing air which has been supplemented to contain a final concentration of 5% carbon dioxide. In addition, the atmosphere should be of a relative humidity greater than 80% and preferably greater than 90%.

The basic constituents of a general tissue culture medium are disclosed by Waymouth, C. (In: Methods for Preparation of Media, Supplements and Substrata for Serum-Free Animal Cell Culture, Alan R. Liss, NY, pp23-68 (1984)) which is hereby incorporated by reference. The basic components of a culture medium include the ions of inorganic salts (such as, for example, $Na^+$, $K^+$, $Ca^{2+}$, $Mg^{2+}$, $Cl^-$, $(HPO_4)^{2-}$ and $(HCO_3)^-$). In addition, a culture medium may contain metal ions (such as, for example, $Fe^{2+}$, $Zn^{2+}$, $Cu^{2+}$, $Mn^{2+}$, $Co^{2+}$ and $(SeO_3)^{2-}$). Additionally, culture media may contain trace amounts of elements such as Sn, V, Cr, Al, As, and Mo.

The culture medium of the invention may also contain amino acids, such as those which naturally occur in animal protein. Vitamins, such as (for example): $B_{12}$, C, A, E, and biotin, as well as lipids and fat soluble components arc also generally included in cultured medium. Lipids and fat soluble components which have been used in culture media include linoleic acid, cholesterol, lecithin, sphingomyelin, phospholipids, and oleic acid, among others.

The culture media of the invention also contain carbohydrates which serve as sources of energy. Although it is preferable to employ glucose and/or glutamine as the principle energy source in the media of the present invention, other sugars (such as fructose, galactose, mannose, maltose or lactose) may also be employed. The concentrations of sugars in such media generally exceeds 1000 mg/L and may exceed 5000 mg/L.

Although the cells of the invention may be capable of synthesizing nucleotides de novo in in vitro, the addition of purine or pyrimidine bases or nucleotides may increase growth efficiency. The bases that may be added to the serum-free media of the invention include adenine and hypoxanthine. Nucleotides include thymidine, deoxyadenosine, deoxycytidine, and deoxyguanosine.

PBL cells are sensitive to changes in the pH of the culture medium. Thus, it is necessary to provide pH buffers to cultured media. Among the buffers which may be used are HEPES, bicarbonate buffers, phosphate buffers, tris buffers, other similar buffers. It is desirable that such buffers be capable of maintaining a pH of approximately 6.5-8.0, and preferably a pH between 6.8 - 7.4.

It is also desirable to supplement the culture media with growth factors, mitogens, or hormones. Among those, growth factors and hormones which may be added include insulin, cortisol or dexamethasone, Interleukin 1, interferons, transferrin, etc. In addition, it is often desirable to include human albumin to the culture medium.

Since PBL cells grow more slowly than potential contaminants, such as bacteria, it may be desirable to supplement the culture medium with antibiotics such as penicillin, streptomycin, gentamycin, etc.

Although it is possible, by routine experimentation, to develop a cell culture medium containing suitable amounts of the above described components, it is alternatively possible to employ any of a wide number of commercially prepared cultural media in which the specific combinations and concentrations of components have already been determined. The compositions of such culture media are disclosed by Freshney, R.I. In: Culture of Animal Cells a Manual of Basic Technique, Alan R. Liss, NY, pp67-78 (1983)).

A "basal medium," as used herein is a medium which contains basic components of the culture medium (such as those described above). Such basal medium (and also the LAK cell-generating medium disclosed herein) is said to be "substantially free of serum" if the concentration of serum is less than 0.1%. Although numerous possible culture media may be formulated which are capable of serving as the basal medium of the present invention, it is especially preferable to employ a basal medium which consists of Dulbecco's Modified Eagle's Medium: Ham's F12 medium in a 1:1, by volume, ratio (Barnes, D. et al., Anal. Biochem. 102:1255 (1980)). Such medium is hereinafter referred to as Darfler's Basal Medium. The compositions of these media are disclosed in Table 1.

## TABLE I

| Component | Dulbecco's modification mg/l | Ham's F12 mg/l | Darfler's Basal Medium mg/l |
|---|---|---|---|
| Inorganic salts | | | |
| $CaCl_2$ (anhyd.) | 200.00 | --- | 100.00 |
| $CaCl_2.2H_2O$ | --- | 44.00 | 22.00 |
| $Fe(NO_3)_3.9H_2O$ | 0.10 | --- | .05 |
| KCl | 400.00 | 223.60 | 311.80 |
| $MgCl_2.6H_2O$ | --- | 122.00 | 61.00 |
| $MgSO_4.7H_2O$ | 200.00 | --- | 100.00 |
| NaCl | 6,400.00 | 7,599.00 | 6,999.50 |
| $NaHCO_3$ | 3,700.00 | 1,176.00 | 2,438.00 |
| $NaH_2PO_4.H_2O$ | 125.00 | --- | 62.50 |
| $NaHPO_4.7H_2O$ | --- | 268.00 | 134.00 |
| $CuSO_4.5H_2O$ | --- | 0.00249 | 0.00125 |
| $FeSO_4.7H_2O$ | --- | 0.834 | 0.417 |
| $ZnSO_4.7H_2O$ | --- | 0.863 | 0.432 |
| Other Components | | | |
| D-glucose | 4,500.00 | 1,802.00 | 3,150.00 |
| Lipoic acid | --- | 0.21 | .11 |
| Phenol red | 15.00 | 12.0 | 13.50 |
| Sodium pyruvate | 110.00 | 110.00 | 110.00 |
| Hypoxanthine | --- | 4.10 | 2.05 |
| Linoleic acid | --- | 0.084 | 0.042 |
| Putrescine 2HCl | --- | 0.161 | 0.081 |
| Thymidine | --- | 0.73 | 0.37 |
| Amino Acids | | | |
| L-alanine | --- | 8.90 | 4.45 |
| L-arginine.HCl | 84.00 | 211.00 | 147.50 |
| $L-asparagine.H_2O$ | --- | 15.01 | 7.50 |
| L-aspartic acid | --- | 13.30 | 6.65 |
| L-cystine | 48.00 | --- | 24.00 |
| $L-cysteine.HCl.H_2O$ | --- | 35.12 | 17.56 |
| L-glutamic acid | --- | 14.70 | 7.35 |
| L-glutamine | 584.00 | 146.00 | 365.00 |
| Glycine | 30.00 | 7.50 | 18.75 |
| $L-histidine\ HCl.H_2O$ | 42.00 | 20.96 | 31.48 |
| L-isoleucine | 105.00 | 3.94 | 54.47 |
| L-leucine | 105.00 | 13.10 | 59.05 |
| L-lysine HCl | 146.00 | 36.50 | 91.25 |
| L-methionine | 30.00 | 4.48 | 17.24 |
| L-phenylalanine | 66.00 | 4.96 | 35.48 |
| L-proline | --- | 34.50 | 17.25 |
| L-serine | 42.00 | 10.50 | 26.25 |
| L-threonine | 95.00 | 11.90 | 53.45 |

7

**TABLE** 1 (cont.d)

| Component | Dulbecco's modification mg/l | Ham's F12 mg/l | Darfler's Basal Medium mg/l |
|---|---|---|---|
| L-tryptophane | 16.00 | 2.04 | 9.02 |
| L-tyrosine | 72.00 | 5.40 | 38.70 |
| L-valine | 94.00 | 11.70 | 52.85 |
| Vitamins | | | |
| Biotin | --- | 0.0073 | .0037 |
| D-Ca pantothenate | 4.00 | 0.4800 | 2.24 |
| Choline chloride | 4.00 | 13.9600 | 8.98 |
| Folic acid | 4.00 | 1.300 | 2.65 |
| i-inositol | 7.20 | 18.000 | 12.60 |
| Nicotinamide | 4.00 | 0.04 | 2.02 |
| Pyridoxal HCl | 4.00 | 0.062 | 2.03 |
| Riboflavin | 0.40 | 0.0380 | .22 |
| Thiamine HCl | 4.00 | 0.3400 | 2.17 |
| Vitamin $B_{12}$ | --- | 1.3600 | .68 |
| Pyridoxine HCl | --- | 0.0620 | .032 |

II. The Generation of Lymphokine-Activated Killer cells.

Cellular immune reactions result from complex interactions between T cells, B cells and macrophages. T cells are "white blood cells" which are formed in the thymus gland. They produce and respond to a variety of different stimuli (such as lymphokines and antigens) to control and modulate an animal's immune response. B cells, which are formed in bone marrow tissue, are the "white blood cells" which produce antibodies in response to infection. Macrophages are the white blood cells which are primarily responsible for removing foreign cells (i.e. bacteria, etc.) from an animal's tissues. The complex interactions between these cells are mediated through direct cellular contact and through soluble compounds known as "lymphokines." Lymphokines are, in general, protein or glycoproteins having a molecular weight between 10-100 kd. Examples of lymphokines are disclosed by Stephenson (In: Animal Cell Biotechnology, Vol. 2, Spier, R.E., et al., eds., Acad. Press, pp 41-45 (1985)). Although any compound capable of directing the activation of PBL cells into LAK cells may be employed according to the present invention, it is preferable to employ a lymphokine, and it is especially preferable to employ the lymphokine, interleukin-2 (IL-2). Interleukin-2 is a 15 kd glycoprotein (Gillis, S., et al., J. Exper. Med. 152:1709 (1980)) which is secreted by activated T lymphocytes (Bonnard, et al., J. Immunol. 123:2704 (1979)). This molecule is believed to interact with a membrane receptor present on activated lymphocytes thereby inducing a cascade phenomena which lead to the proliferation of the activated cells (Robb, J.R., Immunol. Today 7:203 (1984)).

Lymphokines may be purified from natural tissue sources (Spiess, P.J., et al., J. Immunol. Meth. 42:213-222 (1981)), or may be obtained through recombinant DNA techniques (Rosenberg, S.A., et al., Science 223:1412-1414 (1984)) Nishimura, T. et al., Canc. Immunol. Immunother. 21:12 (1986)).

As disclosed above, lymphokines are capable of converting a subset of peripheral blood lymphocytes into LAK cells. Lymphocytes characteristically have a round nucleus with well-condensed chromatin. Methods for preparing and purifying lymphocytes are disclosed by Freshney, R.I. (In: Culture of Animals Cells, A Manual of Basic Technique, Alan R. Liss, Inc., N.Y., p238 (1983) and by Mazumder, A., et al., Cancer, 53:896-905 (1984)).

As indicated, a lymphokine activated killer cell is a peripheral blood lymphocyte cell which has been activated by contact with a lymphokine. Although the precise mechanism through which LAK cells are produced is not fully understood, such an understanding is not necessary in order to practice the invention since it is known to be possible to spontaneously generate LAK cells by incubating PBL cells, under appropriate in vitro tissue culture conditions in the presence of a lymphokine. The amount of lymphokine exogenously added to the culture medium is typically determined by diluting a stock solution of the lymphokine and determining the lowest amount of lymphokine capable of inducing LAK cell formation

8

(Suzuki, R., et al., J. Immunol. 130:981 (1983), Handa, K. et al., J. Immunol. 130:988 (1983)). As little as three units/ml and as much as 1,000 units/ml of interleukin-2 can be used to produce LAK cells from PBL cells. A unit of interleukin-2 is defined as the reciprocal of the dilution that induces 50% of maximal [$^3$H] TdR incorporation of NK-7 cells. (Itoh, K., et al., J. Immunol. 134:1345-1349 (1985)).

A compound is said to be "exogenously added" when its concentration in a cell or medium has been increased through the addition of the compound by one practicing the invention. This addition may either be direct, as by adding the compound itself, or may be indirect, as by adding a precursor of the compound. A compound which is normally and naturally present either within a cell or in a culture medium is not said to be exogenously added to the medium unless its concentration has been increased in a manner consistent with the above definition.

The incubation of PBL cells should be for at least one day (Itoh, K., et al., (1985)), however, the percentage of PBL cells which become activated to LAK cells increases with increasing time. It is preferable to culture PBL cells when they are an initial concentration of between $10^6$ - $8 \times 10^6$ cells/ml. Generally it is not preferable to subculture the cells (i.e. to divide the culture's concentration of cells by adding additional, but sterile, media). It is desirable to maintain LAK-inducing conditions for 3-10 days. Once LAK cells have been produced, the cells may be cultivated in vitro for periods longer than two months (Rayner, A.A., et al., Cancer, 55:1327-1333 (1985)). Under these conditions, it is preferable to subculture LAK cells so as to maintain a cell concentration of between $10^4$ - $10^8$ cells/ml. Since LAK cells, thus expanded, may potentially increase more than $10^{20-}$ fold, LAK cell therapy may be employed even in individuals (such as cancer patients) from whom only small numbers of PBL cells may be obtained. Although interleukin-2 is capable of generating LAK cells without the addition of any other lymphokine, the addition of additional lymphokines (such as gamma interferon) has been found to augment IL-2-induced LAK generation (Shiiba, K., et al., Cancer Immunol. Immunother. 21:119-128 (1986)).

LAK cells are capable of attacking a diverse group of tumors (Grimm, E.A., et al., J. Exper. Med., 155:1823-1841 (1982)). Among the types of tumors thought to be treatable with LAK cell therapy are soft tissue sarcomas, melanomas, osteosarcoma, adenocarcinoma (such as colon, ovarium, and pancreatic carcinomas) lymphomas, esophageal carcinomas and adrenal carcinomas (Rayner, A.A., et al., Cancer 55:1327-1333 (1985)).

An important aspect of the present invention is then discovery that certain non-proteinaceous compounds, capable of activating the endogenous enzyme kinase C, are capable of promoting the generation of LAK cells from PBL cells which have been cultivated in a serum-free medium in the presence of a lymphokine. Such activator compounds are acetylacylglycerols, and diacylglycerols (Ebeling, J.G. Proc. Natl. Acad. Sci. USA, 82:815-819 (1985); Mufson, R.A., Carcinogenesis, 6:1693-1698 (1985)). The synthesis of diacylglycerols which activate kinase C is disclosed by Ebeling, J.G., et al., (1985).

Especially preferred are the diacylglycerols. Although it is possible, according to the present invention, to employ either a racemic mixture of diacylglycerols or one containing unequal amounts of the diacylglycerol's stereoisomers, it is preferable to employ the sn-1,2-diacylglycerols. Especially preferred sn-1-2-diacylglycerols are those with short saturated acyl side chains (containing between 2 and 12, and preferably from between 4 or 6 and 10 carbon atoms, eg sn-1,2-dihexanoylglycerol, sn-1,2-dioctanoylglycerol and sn-1,2-didecanoylglycerol). According to the present invention, the diacylglycerol, sn-1,2-dioctanoylglycerol (diC$_8$), which has an 8 carbon long acyl side chain, was the most preferred reagent for promoting LAK cell generation and proliferation. This compound is capable of mediating such an effect when present at a concentration greater than 1 mg/L, however it is more preferable to employ this reagent at a concentration of from 0.5 or 1.0 to 20 mg/L and most preferable to employ the reagent at a concentration of 5-20 mg/Ll. In general, this compound is only slightly soluble in water (3-5 mg/L). The compound's solubility can, however, be increased to 10-20 mg/L through the use of solubilizing agents such as Tween (at a concentration of between 1-20 mg/L) or Albumin (at a concentration of between 1000-10000 mg/L).

Various methods can be used in order to determine whether a particular compound is an activator of Kinase C. Platelets, for example, can be prelabeled with 32P and then incubated with the compound for 30 seconds. The platelet cells can then be lysed and the proteins liberated from them can be analyzed by sodium dodecyl sulfate-polyacrylamide gel electrophoresis (using, for example, an 11% polyacrylamide gel) and autoradiography. The presence of a radiolabeled 40,000 - dalton protein indicates that the potential activator compound is, indeed, capable of activating kinase C. This method is described by Lapetina, et al. (J. Biol. Chem., 260:135-136 (1985)). Alternatively, the ability of a compound to activate kinase C can be assayed using cell-free kinase C - containing cellular extracts or purified kinase C (in the presence of buffer, MgCl$_2$, CaCl$_2$, H$_1$ Histone (substrate), [gamma - $^{32}$P] ATP and phosphatidyl serine. This method is described by Kraft, A.S. et al. (J. Biol. Chem., 757:13193 (1982)).

Additionally, any analogs of diacylglycerols (such as 1,2-dicaprin, 1,2-dicaproin and 1,2-dicaprylin) or acetylacylglycerols (such as 1-oleoyl-2-acetyl-glycerol) which are capable of activating kinase C may be employed as the activating agent of the present invention.

Having now generally described this invention, the same will be better understood by reference to certain specific examples which are included herein for purposes of illustration only and are not intended to be limiting of the invention, unless specified.

## EXAMPLE I

The Preferred Medium for the Generation of LAK Cells

The Darfler Basal Medium described above and in Table 1 was prepared and supplemented with the following: 50 U/ml penicillin, 50 $\mu$g/ml streptomycin, 10 $\mu$g/ml gentamycin, 3 mg/L ethanolamine, 2 mg/L human insulin, 5 mg/L human transferring, 2500 mg/L human albumin (fraction V or fatty acid-free fraction V), 1 mg/L indomethacin (fatty acid- free), 0.003 mg/L sodium selenite, 2600 mg/L HEPES buffer, (pH 7.0), 2500 mg/L sodium bicarbonate, 3500 mg/L L-glutamine, 55 mg/L pyruvate (sodium salt), 1.5 mg/L cholesterol, 3 mg/L dilinoleoylphosphatidyl choline, 1 mg/L linoleic acid, 1 mg/L palmitic acid, 5mg/L Tween 80 (polyoxyethlenesorbitan, monooleate). Water was added to the medium to bring the osmolality to 280-300 mOsM. Immediately prior to use, 600 U/ml of recombinant interleukin-2 was added to the medium. As used below, when Darfler's Basal Medium is supplemented with all of the above described compounds except for the non-proteinaceous activator of kinase C it is referred to as "Darfler's Supplemented Medium." When the activator compound is added to Darfler's Supplemented Medium the medium is then referred to as "Darfler's Complete Medium" or, alternatively, as Darfler's Supplemented Medium with a disclosed amount of activator compound.

## EXAMPLE II

Cultivation of Peripheral Blood Lymphocytes in a Serum-Free Medium

Human peripheral blood lymphocytes were purified by the method of Mazumder, A. et al. (Cancer, 53: 896-905 (1984)). The cells were cultured at a concentration of $2 \times 10^6$ cells/ml in 2 ml. of Darfler's Complete Medium in 25 cm$^2$ T flasks in a 100% humidity, 5% $CO_2$ environment at 37°C. After 7 days, the cells were counted by hemacytometer and assayed for cytotoxicity against Daudi cells using a Cr release assay according to the method of Mazumder, et al. (1984). By this process a culture of PBL cells was established.

## EXAMPLE III

Diacylglycerol-Containing Serum-Free Medium Allows the Expansion of LAK Cells in Serum-Free Medium in a Seven Day Incubation

PBL cells obtained as described in Example II were grown according to the method of Mazumder, A., et al. (Cancer 53:896-905 (1984)) in RPMI-1640 medium supplemented with 2% human serum or were incubated in either Darfler's Supplemented Medium (described in Example I) or Darfler's Supplemented Medium containing 20 mg/L diC$_8$ and incubated for seven days at 37°C in an atmosphere containing 95% air and 5% $CO_2$. After this incubation, the cytotoxicity of the cultured cells was determined. This experiment revealed that the incubation of PBL cells in serum-free medium which contained a diacylglycerol was able to produce LAK cells at 97% of the efficiency at which such cells were generated after incubation in serum-containing medium. In contrast, serum-free medium which lacked diacylglycerols produced LAK cells at only 1/3 the efficiency of serum-containing medium. These results therefore indicate that a serum-free medium containing diacylglycerols is capable of replacing serum-containing medium for the generation of LAK cells, and that such a serum-free medium is capable of generating LAK cells at an efficiency similar to that of a serum-containing medium.

## EXAMPLE IV

### Amounts of Interleukin-2 Required for LAK Cell Production

PBL cells were prepared as described in Example II and divided into four aliquots which were subsequently incubated in either 98% RPMI-1640 medium supplemented with 2% serum (Mazumder, A., et al. (1984)) or in Darfler's Complete Medium containing the diacylglycerol, sn-1,2-dioctanoylglycerol at 20 mg/L. The Darfler Complete Medium contained either 100, 300, or 1000 U/ml of interleukin-2. After a seven day incubation at 37°C in an atmosphere of 95% air, 5% carbon dioxide the relative efficiency of LAK cells proliferation was determined. Serum-free medium containing 1000 units/ml interleukin-2 was found to produce LAK cells which lysed target cells at 52% the efficiency with which such cells were produced by incubation in serum-containing medium which contained 1000 U/ml interleukin-2. Serum-free medium containing 300 or 100 U/ml interleukin-2 were found, however, to produce LAK cells at an efficiency which was 106% and 85%, respectively, of that observed in the serum-containing medium control. This experiment shows that the use of serum-free medium containing diacylglycerols permits LAK cell formation to occur in the presence of lower amounts of interleukin-2 than are required for LAK cell formation in serum-containing media. Such a result is significant in that the availability and cost of interleukin-2 poses a significant obstacle to the use of LAK-cell therapeutic regimens.

## EXAMPLE V

### Inhibitors of Diacylglycerol Breakdown Enhance LAX Cell Production

PBL cells were prepared as described in Example II and divided into two aliquots which were subsequently incubated in Darfler's Complete Medium (containing sn-1,2-dioctanoylglycerol at 5 mg/L, 1000 U/mL interleukin-2, an additional 10 mg/L Tween 80, and an additional 1.5 mg/L cholesterol) in the presence or absence of 1-monooleoyl-rac-glycerol at 10 mg/L. After seven days incubation at 37°C in an atmosphere of 95% air, 5% carbon dioxide the relative efficiency of LAK cells to lyse target cells was determined. In the absence of 1-monooleoyl-rac-glycerol the percent lysis was 35 at an effector:target of 8:1, 21 at 4:1 and 13 at 2:1. In contrast, in the presence of 1-monooleoyl-rac-glycerol the percent lysis was 50, 33 and 25, respectively. Similar results were obtained in two other experiments, with levels of 1-monooleoyl-rac-glycerol being optimal at 5-10 mg/L. 5 mg/L was found to be the most preferred concentration of monooleoyl-rac-glycerol. Diacyglycerols and their derivatives breakdown due to the action of enzymes such as diacylglycerol kinase and diacylglycerol lipase. Agents (i.e., chemical compounds) which inhibit this breakdown result in higher intercellular levels of diacylglycerols and their derivatives. Since 1-monooleoyl-rac-glycerol inhibits diacylglycerol kinase and diacylglycerol lipase (W.R. Bishop et al., J. Biol. Chem. 261:12513-12519 (1986) and W.R. Bishop et al., J. Biol. Chem., 261:6993-7000 (1986)), these data suggest that the inhibition of these enzymes in cells acts to elevate intracellular levels of diacylglycerol. As a consequence, greater numbers of LAK cells, greater killer activity, or both is generated by the inclusion of 1-monooleoyl-rac-glycerol. Other inhibitors of diacylglycerol breakdown which may have similar activity include, but are not limited to, dioctanoylethylene glycol, dioctanoyl glyceramide, dioctanoyl butanetriol and other analogs of diacylglycerol and monoacylglycerol.

While the invention has been described in connection with specific embodiments thereof, it will be understood that it is capable of further modifications and this application is intended to cover any variations, uses, or adaptations of the invention following, in general, the principles of the invention and including such departures from the present disclosure have come within known or customary practice within the art to which the invention pertains and as may be applied to the essential features herein before set forth and as follows in the scope of the appended claims.

## Claims

1. A method of producing a lymphokine activated killer cell, the method comprising:
   (a) contacting a peripheral blood lymphocyte (PBL) cell with an amount of a culture medium sufficient to permit the in vitro cultivation of the peripheral blood lymphocyte cell, the medium being substantially free of serum and containing:
   (i) a lymphokine; and
   (ii) a non-proteinaceous compound capable of activating the enzyme kinase C which is a diacylglycerol, a functional analogue of a diacylglycerol, or a precursor of a diacylglycerol, an acetylacylglycerol, a functional analogue of an acetylacylglycerol, or a precursor of an acetylacylglycerol; and

(b) maintaining the PBL cell in the culture medium under conditions sufficient to permit the production of the lymphokine activated killer cell.

2. A method according to claim 1 wherein the compound is a diacylglycerol.

3. A method according to Claim 1 or 2 wherein the compound is an sn-1,2-diacylglycerol.

4. A method according to any preceding claim wherein the compound is an sn-1,2-di($C_{6-10}$) acylglycerol.

5. A method according to any preceding claim wherein the compound is sn-1,2-dihexanoylglycerol, sn-1,2-dioctanoylglycerol or sn-1,2-didecanoylglycerol.

6. A method according to any preceding claim wherein the compound is present at from 1.0 to 20 mg/l.

7. A method according to any preceding claim where the compound is present at from 5 to 20 mg/l.

8. A method according to any preceding claim wherein the medium contains an agent capable of inhibiting breakdown of the non-proteinaceous compound.

9. A method according to Claim 8 wherein the agent is an inhibitor of the enzyme diacylglycerol kinase or diacylglycerol lipase.

10. A method according to Claim 8 or 9 wherein the agent is 1-monooleoyl-rac-glycerol, dioctanoylethyleneglycol, dioctanoylglyceramide or dioctanoyl butanetriol.

11. A lymphokine activated killer cell prepared by a process according to any of Claims 1 to 10 for use in medicine.

12. The use of a lymphokine activated killer cell prepared by a process according to any of Claims 1 to 10 in the preparation of an agent for the suppression of growth or development of a cancer cell.

13. A composition comprising:
    (a) an in vitro cultivated peripheral blood lymphocyte cell or a lymphokine activated killer cell;
    (b) an exogenously added non-proteinaceous compound capable of activating the enzyme kinase C which is a diacylglycerol, a functional analogue of a diacylglycerol, or a precursor of a diacylglycerol, an acetylacylglycerol, a functional analogue of an acetylacylglycerol, or a precursor of an acetylacylglycerol; and
    (c) a lymphokine;
wherein the composition is substantially free of serum.

14. A composition according to Claim 13 wherein the compound is a diacylglycerol.

15. A composition according to Claim 13 or 14 wherein the compound is an sn-1,2-di($C_{6-10}$)acylglycerol.

16. A composition according to any of Claims 13 to 15 wherein the compound is sn-1,2-dihexanoylglycerol, sn-1,2-dioctanoylglycerol or sn-1,2-didecanoylglycerol.

17. A culture medium which contains a lymphokine and an exogenously added non-proteinaceous compound capable of activating the enzyme kinase C which is a diacylglycerol, a functional analogue of a diacylglycerol, or a precursor of a diacylglycerol, an acetylacylglycerol, a functional analogue of an acetylacylglycerol, or a precursor of an acetylacylglycerol, and which is substantially free of serum.

18. A composition according to Claim 17 wherein the compound is a diacylglycerol.

19. A composition according to Claim 17 or 18 wherein the compound is an sn-1,2-di($C_{6-10}$)acylglycerol.

12

EP 0 287 652 B1

**20.** A composition according to any of Claims 17 to 19 wherein the compound is sn-1,2-dihexanoylglycerol, sn-1,2-dioctanoylglycerol or sn-1,2-didecanoylglycerol.

**Patentansprüche**

**1.** Verfahren zur Produktion einer Lymphokin-aktivierten Killerzelle, umfassend:

(a) Inkontaktbringen einer Lymphocyten-Zelle von peripherem Blut (PBL) mit einer Menge eines Kulturmediums, die ausreicht, um die in vitro-Züchtung der Lymphocytenzelle von peripherem Blut zu ermöglichen, wobei das Medium im wesentlichen frei von Serum ist und enthält:

(i) ein Lymphokin; und

(ii) eine nicht-proteinartige Verbindung, die zur Aktivierung des Enzyms Kinase C fähig ist, welche ein Diacylglycerin, ein funktionelles Analog eines Diacylglycerins, oder ein Vorläufer eines Diacylglycerins, ein Acetylacylglycerin, ein funktionelles Analog eines Acetylacylglycerins oder ein Vorläufer eines Acetylacylglycerins ist; und

(b) Halten der PBL-Zelle im Kulturmedium unter Bedingungen, die ausreichen, um die Produktion von Lymphokin-aktivierten Killerzellen zu ermöglichen.

**2.** Verfahren nach Anspruch 1, wobei die Verbindung ein Diacylglycerin ist.

**3.** Verfahren nach Anspruch 1 oder 2, wobei die Verbindung ein sn-1,2-Diacylglycerin ist.

**4.** Verfahren nach einem der vorangehenden Ansprüche, wobei die Verbindung ein sn-1,2-Di($C_{6-10}$)-acylglycerin ist.

**5.** Verfahren nach einem der vorangehenden Ansprüche, wobei die Verbindung sn-1,2-Dihexanoylglycerin, sn-1,2-Dioctanoylglycerin oder sn-1,2-Didecanoylglycerin ist.

**6.** Verfahren nach einem der vorangehenden Ansprüche, wobei die Verbindung in einer Menge von 1,0 bis 20 mg/Liter vorliegt.

**7.** Verfahren nach einem der vorangehenden Ansprüche, wobei die Verbindung in einer Menge von 5 bis 20 mg/Liter vorliegt.

**8.** Verfahren nach einem der vorangehenden Ansprüche, wobei das Medium ein Mittel enthält, das zur Hemmung des Abbaus der nicht-proteinartigen Verbindung fähig ist.

**9.** Verfahren nach Anspruch 8, wobei das Mittel ein Inhibitor des Enzyms Diacylglycerinkinase oder Diacylglycerinlipase ist.

**10.** Verfahren nach Anspruch 8 oder 9, wobei das Mittel 1-Monooleoyl-rac-glycerin, Dioctanoylethylenglykol, Dioctanoylglyceramid oder Dioctanoylbutantriol ist.

**11.** Lymphokin-aktivierte Killerzelle, hergestellt durch ein Verfahren nach einem der Ansprüche 1 bis 10 zur Verwendung in der Medizin.

**12.** Verwendung einer Lymphokin-aktivierten Killerzelle, die gemäß einem Verfahren nach einem der Ansprüche 1 bis 10 hergestellt wurde, für die Herstellung eines Mittels zur Unterdrückung des Wachstums oder der Entwicklung einer Krebszelle.

**13.** Mittel enthaltend:

(a) eine in vitro gezüchtete Lymphocytenzelle von peripherem Blut oder eine Lymphokin-aktivierte Killerzelle;

(b) eine exogen zugefügte nicht-proteinartige Verbindung, die zur Aktivierung des Enzyms Kinase C fähig ist, die ein Diacylglycerin, ein funktionelles Analog eines Diacylglycerins oder ein Vorläufer eines Diacylglycerins, ein Acetylacylglycerin, ein funktionelles Analog eines Acetylacylglycerins oder ein Vorläufer eines Acetylacylglycerins ist; und

(c) ein Lymphokin;

wobei das Mittel im wesentlichen frei von Serum ist.

13

**14.** Mittel nach Anspruch 13, wobei die Verbindung ein Diacylglycerin ist.

**15.** Mittel nach Anspruch 13 oder 14, wobei die Verbindung ein sn-1,2-Di($C_{6-10}$)acylglycerin ist.

**16.** Mittel nach einem der Ansprüche 13 bis 16, wobei die Verbindung sn-1,2-Dihexanoylglycerin, sn-1,2-Dioctanoylglycerin oder sn-1,2-Didecanoylglycerin.

**17.** Kulturmedium, enthaltend ein Lymphokin und eine exogen zugesetzte nicht-proteinartige Verbindung, die zur Aktivierung des Enzyms Kinase C fähig ist, die ein Diacylglycerin, ein funktionelles Analog eines Diacylglycerins, oder ein Vorläufer eines Diacylglycerins, ein Acetylacylglycerin, ein funktionelles Analog eines Acetylacylglycerins oder ein Vorläufer eines Acetylacylglycerins ist, das im wesentlichen frei von Serum ist.

**18.** Mittel nach Anspruch 17, wobei die Verbindung ein Diacylglycerin ist.

**19.** Mittel nach Anspruch 17 oder 18, wobei die Verbindung ein sn-1,2-Di($C_{6-10}$)acylglycerin ist.

**20.** Mittel nach einem der Ansprüche 17 bis 19, wobei die Verbindung sn-1,2-Dihexanoylglycerin, sn-1,2-Dioctanoylglycerin oder sn-1,2-Didecanoylglycerin ist.

**Revendications**

**1.** Procédé de production d'une cellule tueuse activée par une lymphokine, le procédé comprenant :
(a) la mise en contact d'un lymphocyte du sang périphérique (peripheral blood lymphocyte - PBL) avec une quantité d'un milieu de culture suffisante pour permettre la culture in vitro du lymphocyte du sang périphérique, le milieu étant sensiblement exempt de sérum et contenant:
(i) une lymphokine ; et
(ii) un composé non-protéinique apte à activer l'enzyme kinase C, qui est un diacyl glycérol, un analogue fonctionnel d'un diacyl glycérol, ou un précurseur d'un diacyl glycérol, un acétyl acyl glycérol, un analogue fonctionnel d'un acétyl acyl glycérol, ou un précurseur d'un acétyl acyl glycérol ; et
(b) le maintien de la cellule PBL dans le milieu de culture dans des conditions suffisantes pour permettre la production de la cellule tueuse activée par la lymphokine.

**2.** Procédé selon la revendication 1, dans lequel le composé est un diacyl glycérol.

**3.** Procédé selon la revendication 1 ou 2, dans lequel le composé est un sn-1,2-diacyl glycérol.

**4.** Procédé selon l'une des revendications précédentes, dans lequel le composé est un sn-1,2-di($C_{6-10}$)-acyl glycérol.

**5.** Procédé selon l'une des revendications précédentes, dans lequel le composé est le sn-1,2-dihexanoyl glycérol, le sn-1,2-dioctanoyl glycérol ou le sn-1,2-didécanoyl glycérol.

**6.** Procédé selon l'une des revendications précédentes, dans lequel le composé est présent à raison de 1,0 à 20 mg/l.

**7.** Procédé selon l'une des revendications précédentes, dans lequel le composé est présent à raison de 5 à 20 mg/l.

**8.** Procédé selon l'une des revendications précédentes, dans lequel le milieu contient un agent apte à inhiber la dégradation du composé non-protéinique.

**9.** Procédé selon la revendication 8, dans lequel l'agent est un inhibiteur de l'enzyme diacyl glycérol kinase ou diacyl glycérol lipase.

**10.** Procédé selon la revendication 8 ou 9, dans lequel l'agent est le 1-monooléoyl-rac-glycérol, le dioctanoyl éthylène glycol, le dioctanoyl glycéramide ou le dioctanoyl butane triol.

11. Cellule tueuse activée par une lymphokine, préparée par un procédé tel que défini à l'une des revendications 1 à 10, pour l'utilisation en médecine.

12. Utilisation d'une cellule tueuse activée par une lymphokine, préparée par un procédé tel que défini à l'une des revendications 1 à 10, dans la préparation d'un agent pour la suppression de la croissance ou du développement d'une cellule cancéreuse.

13. Composition comprenant :

(a) un lymphocyte du sang périphérique cultivé in vitro ou une cellule tueuse activée par une lymphokine ;

(b) un composé non-protéinique ajouté de façon exogène apte à activer l'enzyme kinase C, qui est un diacyl glycérol, un analogue fonctionnel d'un diacyl glycérol, ou un précurseur d'un diacyl glycérol, un acétyl acyl glycérol, un analogue fonctionnel d'un acétyl acyl glycérol, ou un précurseur d'un acétyl acyl glycérol ; et

(c) une lymphokine ;

où la composition est sensiblement exempte de sérum.

14. Composition selon la revendication 13, dans laquelle le composé est un diacyl glycérol.

15. Composition selon la revendication 13 ou 14, dans laquelle le composé est un sn-1,2-di($C_{6-10}$)acyl glycérol.

16. Composition selon l'une des revendications 13 à 15, dans laquelle le composé est le sn-1,2-dihexanoyl glycérol, le sn-1,2-dioctanoyl glycérol ou le sn-1,2-didécanoyl glycérol.

17. Milieu de culture qui contient une lymphokine et un composé non-protéinique ajouté de façon exogène apte à activer l'enzyme kinase C, qui est un diacyl glycérol, un analogue fonctionnel d'un diacyl glycérol, ou un précurseur d'un diacyl glycérol, un acétyl acyl glycérol, un analogue fonctionnel d'un acétyl acyl glycérol, ou un précurseur d'un acétyl acyl glycérol, et qui est sensiblement exempt de sérum.

18. Composition selon la revendication 17, dans laquelle le composé est un diacyl glycérol.

19. Composition selon la revendication 17 ou 18, dans laquelle le composé est un sn-1,2-di($C_{6-10}$)acyl glycérol.

20. Composition selon l'une des revendications 17 à 19, dans laquelle le composé est le sn-1,2-dihexanoyl glycérol, le sn-1,2-dioctanoyl glycérol ou le sn-1,2-didécanoyl glycérol.